# EUROPEAN PATENT APPLICATION

(11) **EP 2 425 874 A2**
(43) Date of publication of application: **07.03.2012**
(21) Application number: 11175046.9
(22) Date of filing: 23.08.2007
(51) Int. Cl.: A61P 35/00, A61K 31/337, A61K 31/59, A61K 31/573

(54) **Use of a vitamin D compound and an additional therapeutic agent for the treatment of cancer**

(30) Priority: 25.08.2006 US 934924 P
(62) Divisional of application: 07837327.1
(71) Applicant: Cougar Biotechnology, Inc., Los Angeles CA 90024 (US)
(72) Inventor: Auerbach, Alan, H., Los Angeles, CA California 90024 (US); Belldegrun, Arie, S., Los Angeles, CA California 90024 (US)
(74) Representative: Warner, James Alexander

(57) **Abstract**

Methods and compositions for treating cancer are described herein. More particularly, the methods for treating cancer comprise administering a vitamin D compound in combination with at least one additional therapeutic agent, such as an anti-cancer agent or a steroid. Furthermore, disclosed are compositions comprising vitamin D compounds and at least one additional therapeutic agent, such as an anti-cancer agent or a steroid, e.g., corticosteroid or more specifically, a glucocorticoid.

## Description

### FIELD OF THE INVENTION

Methods and compositions for treating cancer are described herein. More particularly, the methods for treating cancer comprise administering a vitamin D compound in combination with at least one additional therapeutic agent, such as an anti-cancer agent or a steroid. Furthermore, disclosed are compositions comprising vitamin D compounds and at least one additional therapeutic agent, such as an anti-cancer agent or a steroid, e.g., corticosteroid or, more specifically, a glucocorticoid.

### BACKGROUND

Currently, cancer is the second leading case of death in the United States after heart disease. Incidences of cancer are widely expected to increase as the U.S. population ages and, if current trends continue, cancer is expected to be the leading cause of the death by the year 2010. As of now, it is estimated that one in every two men in the United States will be diagnosed with cancer at some time during his lifetime and one in three women in the United States will be diagnosed with cancer at some time during her lifetime. Of special interest, are individuals diagnosed with prostate cancer and breast cancer since, along with lung cancer, prostate cancer and breast cancer are the most common and fatal forms of cancer.

Prostate cancer is currently the most common non-skin cancer and the second leading cause of cancer-related death in men after lung cancer. The primary course of treatment for patients diagnosed with organ-confined prostate cancer is usually prostatectomy or radiotherapy. Not only are these treatments highly invasive and have undesirable side effects, such localized treatments are not effective on prostate cancer after it has metastasized. Moreover, a large percent of individuals who receive localized treatments will suffer from recurring cancer.

Additionally, breast cancer is the most common cancer among white and African-American women. Similar to treating prostate cancer, most options for women diagnosed with breast cancer are highly invasive and have significant side-effects. Such treatments include surgery, radiation and chemotherapy.

Another treatment option for individuals with cancer comprises administering vitamin D alone or in combination with chemotherapy or radiotherapy treatments. Vitamin D has been found to inhibit cell growth and stimulate cell differentiation in several *in vitro* and *in vivo* cancer models.

However, a major limitation of using vitamin D as a therapeutic agent is its tendency to cause hypercalcemia. Hypercalcemia is a higher than normal level of calcium in the blood that can cause nausea, vomiting, confusion, lethargy, fatigue, excessive thirst, abdominal pain, constipation and muscle weakness. In fact, the use of vitamin D in clinical trials has been hampered by its ability to induce hypercalcemia at serum concentrations required to suppress cancer cell proliferation.

Despite the progress made in the treatment of cancer, there remains a need for more effective ways to treat cancer such as, but not limited to, prostate cancer and breast cancer that do not include the side effects caused by current cancer treatments. Additionally, there is a need for effective anti-cancer treatment options for patients who are not responding to current anti-cancer treatments.

### SUMMARY OF THE INVENTION

Described herein are methods for treating a cancer in which a therapeutically effective amount of a vitamin D compound, such as seocalcitol, is administered to a patient, e.g., a patient in need thereof, in combination with a therapeutically effective amount of at least one additional therapeutic agent including, but not limited to, an anti-cancer agent or steroid. Such methods also provide an effective treatment for individuals with a refractory cancer, including individuals who are currently undergoing a cancer treatment. Therefore, in certain embodiments, the method is directed to treating a refractory cancer in a patient in which a therapeutically effective amount of a vitamin D compound, such as seocalcitol, is administered to a patient currently receiving another cancer treatment, e.g., the administration of an additional anti-cancer agent. Preferably, the vitamin D compound is a non-hypercalcemic vitamin D compound analog, such as seocalcitol. Also, the vitamin D compound can be administered by pulse administration.

For example, in certain embodiments, the method is directed to the treatment of a cancer in a mammal by administering an amount of about 0.1 µg/day to about 500 µg/day of seocalcitol, such as about 100 µg/day of seocalcitol, and an amount of about 0.1 mg/m² to about 20 mg/m² of mitoxantrone.

In another embodiment, the method is directed to the treatment of a cancer in a mammal by administering an amount of about 0.1 µg/day to about 500 µg/day of seocalcitol, such as about 100 µg/day of seocalcitol, and an amount of about 1 mg/m² to about 175 mg/m² of paclitaxel.

In still other embodiments, the method is directed to the treatment of a cancer in a mammal by administering an amount of about 0.1 µg/day to about 500 µg/day of seocalcitol, such as about 100 µg/day of seocalcitol, and an amount of about 1 mg/m² to about 100 mg/m² of docetaxel.

The method for the treatment of a cancer in a mammal can also comprise administering an amount of about 0.1 µg/day to about 500 µg/day of seocalcitol, such as about 100 µg/day of seocalcitol, and an amount of about 0.01 mg to about 200 mg of leuprolide, wherein the leuprolide is administered over a period of about 3 days to about 12 months.

The method can also comprise administering an amount of about 0.1 µg/day to about 500 µg/day of seocalcitol, such as about 100 µg/day of seocalcitol, and an amount of about 0.01 mg to about 20 mg of goserelin, wherein the goserelin is administered over a period of about 28 days to about 3 months.

Additionally, the treatment of a cancer in a mammal can comprise administering an amount of about 0.1 µg/day to about 500 µg/day of seocalcitol, such as about 100 µg/day of seocalcitol, and an amount of about 0.01 mg to about 20 mg of triptorelin, wherein the triptorelin is administered over a period of about 1 month.

In some embodiments, the method for the treatment of a cancer in a mammal comprises administering an amount of about 0.1 µg/day to about 500 µg/day of seocalcitol, such as about 100 µg/day of seocalcitol, and an amount of about 0.4 mg/day to about 10,000 mg/day of abiraterone acetate.

In other embodiments, the method for the treatment of a cancer in a mammal comprises administering an amount of about 0.1 µg/day to about 500 µg/day of seocalcitol, such as about 100 µg/day of seocalcitol, and an amount of about 1 mg/day to about 300 mg/day of bicalutamide.

Also, the method for the treatment of a cancer in a mammal can comprise administering an amount of about 0.1 µg/day to about 500 µg/day of seocalcitol, such as about 100 µg/day of seocalcitol, and an amount of about 1 mg/day to about 2000 mg/day of flutamide.

Moreover, the method for the treatment of a cancer in a mammal can comprise administering an amount of about 0.1 µg/day to about 500 µg/day of seocalcitol, such as about 100 µg/day of seocalcitol, and an amount of about 0.01 mg/day to about 500 mg/day of a glucocorticoid including, but not limited to, hydrocortisone, prednisone or dexamethasone.

Also described herein are compositions for the treatment of cancer that comprise a combination of a therapeutically effective amount of a vitamin D compound, such as seocalcitol, and a therapeutically effective amount of an additional anti-cancer agent, such as, but not limited to, mitoxantrone, paclitaxel, docetaxel, leuprolide, goserelin, triptorelin, abiraterone acetate, bicalutamide, flutamide or a steroid, including, but not limited to, hydrocortisone, prednisone or dexamethasone.

Finally, single unit dosage forms comprising a vitamin D compound, such as seocalcitol, and a glucocorticoid, optionally with carriers, diluents or excipients, are contemplated. Also, kits comprising at least one vitamin D compound and an additional anti-cancer agent or steroid are contemplated. For example, the kit may include a vial containing seocalcitol and another vial containing a glucocorticoid.

### Definitions

As used herein and unless otherwise defined the word "cancer," refers to the growth, division or proliferation of abnormal cells in the body. Cancers that can be treated with the methods and the compositions described herein include, but are not limited to, prostate cancer, breast cancer, adrenal cancer, leukemia, lymphoma, myeloma, Waldenström's macroglobulinemia, monoclonal gammopathy, benign monoclonal gammopathy, heavy chain disease, bone and connective tissue sarcoma, brain tumors, thyroid cancer, pancreatic cancer, pituitary cancer, eye cancer, vaginal cancer, vulvar cancer, cervical cancer, uterine cancer, ovarian cancer, esophageal cancer, stomach cancer, colon cancer, rectal cancer, liver cancer, gallbladder cancer, cholangiocarcinoma, lung cancer, testicular cancer, penal cancer, hepatocellular cancer, oral cancer, skin cancer, kidney cancers, Wilms' tumor and bladder cancer.

As used herein, and unless otherwise defined, the terms "treat," "treating" and "treatment" include the eradication, removal, modification, management or control of a tumor or primary, regional, or metastatic cancer cells or tissue and the minimization or delay of the spread of cancer.

As used herein, and unless otherwise defined, the term "patient" means an animal, including but not limited to an animal such as a human, monkey, cow, horse, sheep, pig, chicken, turkey, quail, cat, dog, mouse, rat, rabbit, or guinea pig. In one embodiment, the patient is a mammal and in another embodiment the patient is a human. In certain embodiments, the patient can be an adult male or female. In some embodiments, the patient is a male of age about 30 years to about 85 years. In other embodiments, the patient is a female of age about 30 years to about 85 years. In a particular embodiment, the patient has or is susceptible to having (e.g., through genetic or environmental factors) cancer. In a further embodiment, the patient has or is susceptible to having (e.g., through genetic or environmental factors) a tumor. In other embodiments, the patient can be castrated or non-castrated.

The term *"vitamin D compound"* as used herein refers to vitamin D; metabolites of vitamin D, such as 1α,25-dihydroxycholecalciferol, as well as derivatives, analogs and pharmaceutically acceptable salts of vitamin D.

The phrase "non-hypercalcemic vitamin D compound" refers to a vitamin D compound that at least gives rise to reduced incidences or reduced severity of hypercalcemia as compared to the active form of vitamin D.

The phrase "non-hypercalcemic vitamin D analog" refers to an analog of vitamin D that at least gives rise to reduced incidences or reduced severity of hypercalcemia as compared to the active form of vitamin D.

The phrases "pulse administration of a vitamin D compound" or "pulse dosing of a vitamin D compound" refers to a dosing regimen of a vitamin D compound that is interrupted by periods of non-dosing such that the dosing regimen allows the vitamin D compound to have a therapeutic effect while avoiding incidences hypercalcemia. The periods of non-dosing can be from three days to one month. For example, a pulse administration dosing regimen can include a single dose administration of a vitamin D compound followed by three weeks of non-administration of a vitamin D compound.

The phrase "anti-cancer agent" as used herein refers to any therapeutic agent that directly or indirectly kills cancer cells or directly or indirectly prohibits stops or reduces the proliferation of cancer cells. It should be noted that even though throughout this specification and in the claims the phrase "anti-cancer agent" is written as a singular noun, for example; "an anti-cancer agent" or "the anti-cancer agent," the phrase "anti-cancer agent" should not be interpreted as being limited to the inclusion of a single anti-cancer agent.

The term "17α-hydroxylase/C_{17,20}-lyase inhibitor" as used herein refers to an inhibitor of 17α-hydroxylase/C_{17,20}-lyase, (which is an enzyme in testosterone synthesis), an analog thereof, derivative thereof, metabolite thereof or pharmaceutically acceptable salt thereof. Also, unless otherwise noted, reference to a particular 17α-hydroxylase/C_{17,20}-lyase inhibitor can include analogs, derivatives, metabolites or pharmaceutically acceptable salts of such particular 17α-hydroxylase/C_{17,20}-lyase inhibitor.

As used herein, and unless otherwise defined, the phrase "therapeutically effective amount" when used in connection with a vitamin D compound or therapeutic agent means an amount of the vitamin D compound or therapeutic agent effective for treating a disease or disorder disclosed herein, such as cancer.

As used herein and unless otherwise defined the phrase "refractory cancer," means cancer that is not responding to an anti-cancer treatment or cancer that is not responding sufficiently to an anti-cancer treatment. Refractory cancer can also include recurring or relapsing cancer.

As used herein and unless otherwise defined the phrase "refractory patient," means a patient who has refractory cancer.

As used herein and unless otherwise defined the phrase "relapse cancer," means cancer that was at one time responsive to an anti-cancer treatment but has become no longer responsive to such treatment or is no longer responding sufficiently to such treatment.

As used herein and unless otherwise defined the phrase "recurring cancer," means cancer that has returned after a patient has been earlier diagnosed with cancer, under gone treatment or had been previously diagnosed as cancer-free.

As used herein and unless otherwise defined the term "derivative" refers to a chemically modified compound wherein the chemical modification takes place at one or more functional groups of the compound. The derivative may retain or improve the pharmacological activity of the compound from which it is derived.

As used herein and unless otherwise defined the term "analog" refers to a chemical compound that is structurally similar to another but differs slightly in composition (as in the replacement of one atom by an atom of a different element or in the presence of a particular functional group).

As used herein and unless otherwise defined the phrase "pharmaceutically acceptable salt" refers to any salt of a chemical compound that retains the biological effectiveness of the compound. Examples of pharmaceutically acceptable salts include, but are not limited to, acetates, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phylacetates, phenylpropionates, phenylbutyrates, citrates, lactates, gamma-hydroxybutyrates, glycollates, tartarates, alkanesulfonates (e.g. methane-sulfonate or mesylate), propanesulfonates, naphthalene-1-sulfonates, naphthalene-2-sulfonates, and mandelates. Several of the officially approved salts are listed in Remington: The Science and Practice of Pharmacy, Mack Publ. Co., Easton.

### DETAILED DESCRIPTION OF THE INVENTION

The methods described herein for treating cancer comprise administering to a mammal, preferably a human, a vitamin D compound, such as seocalcitol, in addition to at least one therapeutic agent, such as an anti-cancer agent or a steroid, particularly a corticosteroid or a glucocorticoid. The compositions described herein comprise a vitamin D compound and at least one additional therapeutic agent, such as an anti-cancer agent or a steroid, particularly a glucocorticoid. Other anti-cancer treatments such as, administration of another anti-cancer agent, radiotherapy, chemotherapy, photodynamic therapy, surgery or other immunotherapy, can be used with the methods and compositions.

### Vitamin D Compounds

Vitamin D is a fat soluble vitamin which is essential as a positive regulator of calcium homeostasis. The active form of vitamin D is 1α,25-dihydroxyvitamin D₃, also known as calcitriol. In addition to influencing calcium homeostasis, many reports demonstrate the utility of active vitamin D compounds in the treatment of cancer.

Vitamin D compounds suitable for the methods and compositions include, but are not limited to, vitamin D, calcitriol, 1α-hydroxy derivatives with a 17 side chain greater in length than the cholesterol or ergosterol side chains (see U.S. Patent No. 4,717,721); cyclopentano-vitamin D analogs (see U.S. Patent No. 4,851,401); vitamin D₃ analogues with alkynyl, alkenyl, and alkanyl side chains (see U.S. Patent Nos. 4,866,048 and 5,145,846); trihydroxycalciferol (see U.S. Patent No. 5,120,722); fluoro-cholecalciferol compounds (see U.S. Patent No. 5,547,947); methyl substituted vitamin D (see U.S. Patent No. 5,446,035); 23-oxa-derivatives (see U.S. Patent No. 5,411,949); 19-nor-vitamin D compounds (see U.S. Patent No. 5,237,110); and hydroxylated 24-homo-vitamin D derivatives (see U.S. Patent No. 4,857,518). Particular examples include ROCALTROL (Roche Laboratories); CALCIJEX injectable calcitriol; seocalcitol; 24a,26a,27a-trihomo-22,24-diene-1αa,25-(OH)₂-D₃; 20-epi-22-oxa-24a,26a,27a-trihomo-1α,25-(OH)₂- D₃, 1,25-(OH)₂-20-epi-D₃); calcipotriol, 1α,24s-(OH)₂-22-ene-26,27-dehydro-D₃,); Roche Pharmaceutical drugs that include 1,25-(OH)₂-16-ene-D₃, 1,25-(OH)₂-16-ene-23-yne- D₃, and 25-(OH)₂-16-ene-23-yne-D₃; Chugai Pharmaceuticals 22-oxacalcitriol (22-oxa-1α,25-(OH)₂ - D₃; 1α-(OH)-D₅ from the University of Illinois; and drugs from the Institute of Medical Chemistry-Schering AG that include ZK 161422 (20-methyl-1,25-(OH)₂- D₃) and ZK 157202 (20-methyl-23-ene-1,25-(OH)₂- D₃); 1α-(OH)-D₂; 1α-(OH)- D₃ and 1α-(OH)-D₄.

Of special interest are non-hypercalcemic vitamin D compounds, such as non-hypercalcemid vitamin D analogs. Examples of non-hypercalcemic vitamin D compounds have the structure of formula (I): 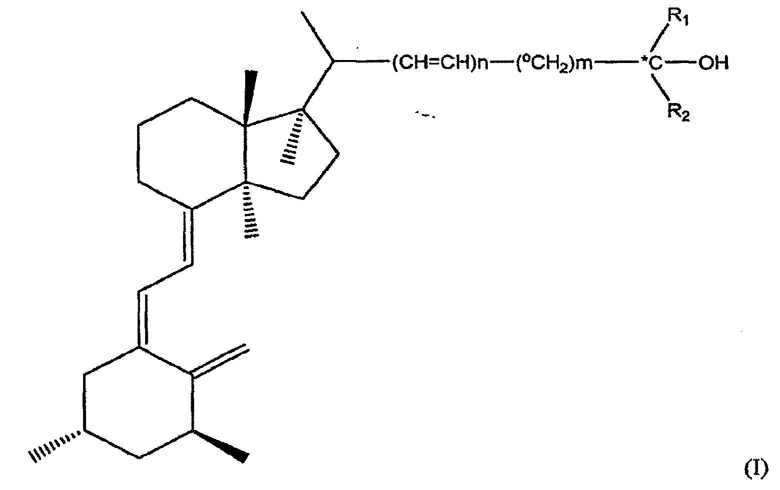 wherein n is 2 or 3, m is 0 or an integer from 1 to 4; R¹ and R² (which may be the same or different) stand for hydrogen or C₁-C₈, hydrocarbyl indicating the residue after removal of a hydrogen atom from a straight, branched or cyclic saturated or unsaturated hydrocarbon, or taken together with the carbon bearing the hydroxyl group (starred in formula I), R¹ and R² can form a saturated or unsaturated C₃-C₈ carbocyclic ring; in addition, R¹ and/or R² and/or one of the m carbons designated by the "*" may be optionally substituted with one or more chlorine or fluorine atom(s); and finally one of the carbons designated "°" may optionally be substituted by one or two C₁-C₂ alkyl group(s); and derivatives of the compounds of formula (1) in which one or more hydroxy have been transformed into -O-acyl or -O-glycosyl or phosphate ester groups; such masked groups being hydrolyzable in vivo; and other prodrugs thereof. The formula (I) and compounds of formula (I) were disclosed in United States Patent No. 5,190,935 to Binderup et al.*,* herein incorporated by reference in its entirety.

Other examples of non-hypercalcemic vitamin D compounds have the structure of formula (II): 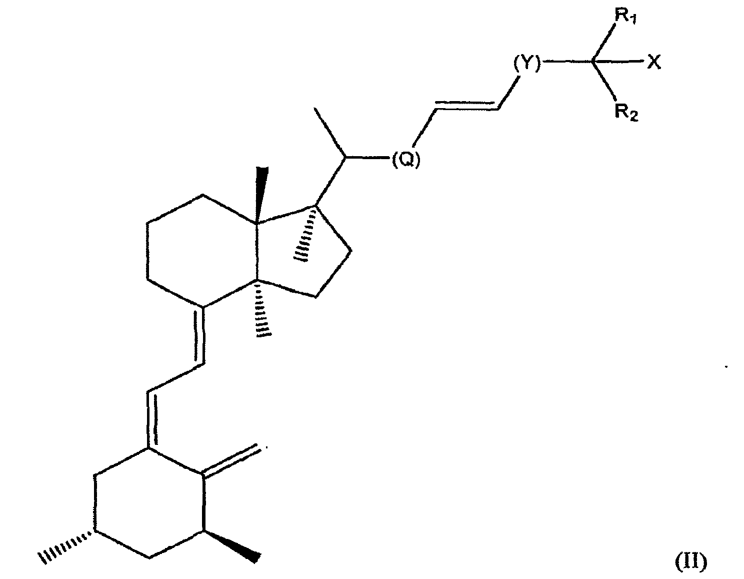 wherein X is hydrogen or hydroxy; R¹ and R² stand for methyl or ethyl (in which R¹ and R² can be the same or different); Q is methylene, ethylene, tri- or tetra-methylene and may optionally be substituted with an oxy group, --OR³, in which R³ is hydrogen, methyl or ethyl; Y is either a single bond or C₁-C₂ hydrocarbylene where hydrocarbylene is the diradical obtained after removal of two hydrogen atoms from a straight, branched or cyclic, saturated or unsaturated hydrocarbon and R¹, R² or Y is unsubstituted or substituted by one or more fluorine atoms or a hydroxyl group. The formula (II) and compounds of formula (II) were disclosed in United States Patent No. 6,310,226 to Calverley et al.*,* herein incorporated by reference in its entirety.

In a preferred embodiment, the vitamin D compound is the non-hypercalcemic vitamin D analog, seocalcitol or 1(S),3(R)-dihydroxy-20(R)-(5'-ethyl-5'-hydroxyhepta-1'(E),3'(E)-dien-1'-yl)-9,10-secopregna-5(Z),7(E),-10(19)-triene which has the following structural formula:

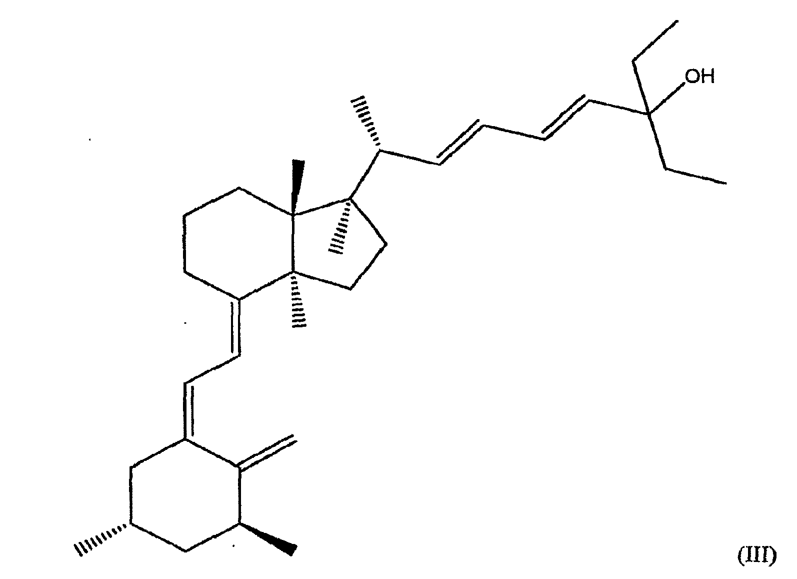

Seocalcitol has displayed strong anti-proliferative activity, low calcemic activity and a biological half-life suitable for systematic use. Seocalcitol has been found to be 50-200 times more potent in regulation of cell growth and differentiation than 1α,25-dihydroxycholecalciferol in vitro and in vivo, however, its calcemic activity was approximately 50% weaker than that of 1α,25-dihydroxycholecalciferol in vivo.

Non-hypercalcemic vitamin D compounds can be made according to any method known to one skilled in the art. An example of a method of synthesizing non-hypercalcemic analogs of vitamin D was described in Hansen et al., Current Pharmaceutical Design, 6, 803-828, 2000, herein incorporated by reference in its entirety. An additional method of synthesizing seocalcitol was described in Sabroe et al., Org. Proc. Res. Dev., 8(1), 133-135, 2004 and Posner et al., Bioorg. Med. Chem., 9(9), 2365-71, 2001, herein incorporated by reference in its entirety.

The therapeutically effective amount of the vitamin D compound administered to a mammal having cancer is an amount that is sufficient to treat the cancer, whether the vitamin D compound is administered alone or in combination with an additional anti-cancer treatment, such as an additional anti-cancer agent.

### Additional Therapeutic Agents

Suitable compounds that can be used in addition to a vitamin D compound as an anti-cancer agent include, but are not limited to, hormone ablation agents, anti-androgen agents, differentiating agents, anti-neoplastic agents, kinase inhibitors, anti-metabolite agents, alkylating agents, antibiotic agents, immunological agents, interferon-type agents, intercalating agents, growth factor inhibitors, cell cycle inhibitors, enzymes, topoisomerase inhibitors, biological response modifiers, mitotic inhibitors, matrix metalloprotease inhibitors, genetic therapeutics, testosterone synthesis inhibitors, and anti-androgens. The amount of the additional anti-cancer agent administered to a mammal having cancer is an amount that is sufficient to treat the cancer whether administered alone or in combination with a vitamin D compound. Below are examples of some of the above classes of anti-cancer agents. The examples are not all inclusive and are for purposes of illustration and not for purposes of limitation. Many of the examples below could be listed in multiple classes of anti-cancer agents and are not restricted in any way to the class in which they are listed in.

Suitable hormonal ablation agents include, but are not limited to, androgen ablation agents and estrogen ablation agents. In preferred embodiments, the vitamin D compound is administered with a hormonal ablation agent, such as deslorelin, leuprolide, goserelin, 17α-hydroxylase/C₁₇, ₂₀-lyase inhibitor, such as abiraterone acetate (*i*.*e*. 3β-acetoxy-17-(3-pyridyl) androsta-5, 16-diene) (*see e.g.,* U.S. Patent No. 5,604,213 to Barrie et al.), or triptorelin. Even though throughout this specification and in the claims the phrase "hormonal ablation agent" is written as a singular noun, for example; "a hormonal ablation agent" or "the hormonal ablation agent," the phrase "hormonal ablation agent" should not be interpreted as being limited to the inclusion of a single hormonal ablation agent. The amount of the hormonal ablation agent administered to a mammal having cancer is an amount that is sufficient to treat the cancer whether administered alone or in combination with a vitamin D compound.

Suitable anti-androgen agents include but are not limited to bicalutamide, flutamide and nilutamide. The amount of the anti-androgen agent administered to a mammal having cancer is an amount that is sufficient to treat the cancer whether administered alone or in combination with a vitamin D compound.

In another embodiment, the vitamin D compound may be administered with a differentiating agent. Suitable differentiating agents include, but are not limited to, polyamine inhibitors; additional vitamin D compounds; metabolites of vitamin A, such as, ATRA, retinoic acid, retinoids; short-chain fatty acids; phenylbutyrate; and nonsteroidal anti-inflammatory agents. The amount of the differentiating agent administered to a mammal having cancer is an amount that is sufficient to treat the cancer whether administered alone or in combination with a vitamin D compound.

In another preferred embodiment, the vitamin D compound may be administered with an anti-neoplastic agent, including, but not limited to, tubulin interacting agents, topoisomerase inhibitors and agents, acitretin, alstonine, amonafide, amphethinile, amsacrine, ankinomycin, anti-neoplaston, aphidicolin glycinate, asparaginase, baccharin, batracylin, benfluron, benzotript, bromofosfamide, caracemide, carmethizole hydrochloride, chlorsulfaquinoxalone, clanfenur, claviridenone, crisnatol, curaderm, cytarabine, cytocytin, dacarbazine, datelliptinium, dihaematoporphyrin ether, dihydrolenperone, dinaline, distamycin, docetaxel, elliprabin, elliptinium acetate, epothilones, ergotamine, etoposide, etretinate, fenretinide, gallium nitrate, genkwadaphnin, hexadecylphosphocholine, homoharringtonine, hydroxyurea, ilmofosine, isoglutamine, isotretinoin, leukoregulin, lonidamine, merbarone, merocyanine derivatives, methylanilinoacridine, minactivin, mitonafide, mitoquidone, mitoxantrone, mopidamol, motretinide, N-(retinoyl)amino acids, N-acylated-dehydroalanines, nafazatrom, nocodazole derivative, ocreotide, oquizanocine, paclitaxel, pancratistatin, pazelliptine, piroxantrone, polyhaematoporphyrin, polypreic acid, probimane, procarbazine, proglumide, razoxane, retelliptine, spatol, spirocyclopropane derivatives, spirogermanium, strypoldinone, superoxide dismutase, teniposide, thaliblastine, tocotrienol, topotecan, ukrain, vinblastine sulfate, vincristine, vindesine, vinestramide, vinorelbine, vintriptol, vinzolidine, and withanolides. The amount of the anti-neoplastic agent administered to a mammal having cancer is an amount that is sufficient to treat the cancer whether administered alone or in combination with a vitamin D compound.

The vitamin D compound may also be used with a kinase inhibitor including p38 inhibitors and CDK inhibitors, TNF inhibitors, metallomatrix proteases inhibitors (MMP), COX-2 inhibitors including celecoxib, rofecoxib, parecoxib, valdecoxib, and etoricoxib, SOD mimics or αᵥβ₃ inhibitors. The amount of the kinase inhibitor administered to a mammal having cancer is an amount that is sufficient to treat the cancer whether administered alone or in combination with a vitamin D compound.

In another embodiment, vitamin D compound may be administered with an anti-metabolite agent. Suitable anti-metabolite agents may be selected from, but not limited to, 5-FU-fibrinogen, acanthifolic acid, aminothiadiazole, brequinar sodium, carmofur, cyclopentyl cytosine, cytarabine phosphate stearate, cytarabine conjugates, dezaguanine, dideoxycytidine, dideoxyguanosine, didox, doxifluridine, fazarabine, floxuridine, fludarabine phosphate, 5-fluorouracil, N-(2'-furanidyl)-5-fluorouracil, isopropyl pyrrolizine, methobenzaprim, methotrexate, norspermidine, pentostatin, piritrexim, plicamycin, thioguanine, tiazofurin, trimetrexate, tyrosine kinase inhibitors, and uricytin. The amount of the anti-metabolite agent administered to a mammal having cancer is an amount that is sufficient to treat the cancer whether administered alone or in combination with a vitamin D compound.

In another embodiment, the vitamin D compound may be administered with an alkylating agent. Suitable alkylating agents may be selected from, but not limited to, aldo-phosphamide analogues, altretamine, anaxirone, bestrabucil, budotitane, carboplatin, carmustine, chlorambucil, cisplatin, cyclophosphamide, cyplatate, diphenylspiromustine, diplatinum cytostatic, elmustine, estramustine phosphate sodium, fotemustine, hepsul-fam, ifosfamide, iproplatin, lomustine, mafosfamide, mitolactol, oxaliplatin, prednimustine, ranimustine, semustine, spiromustine, tauromustine, temozolomide, teroxirone, tetraplatin and trimelamol. The amount of the alkylating agent administered to a mammal having cancer is an amount that is sufficient to treat the cancer whether administered alone or in combination with a vitamin D compound.

In another preferred embodiment, the vitamin D compound may be administered with an antibiotic agent. Suitable antibiotic agents may be selected from, but not limited to, aclarubicin, actinomycin D, actinoplanone, adriamycin, aeroplysinin derivative, amrubicin, anthracycline, azino-mycin-A, bisucaberin, bleomycin sulfate, bryostatin-1, calichemycin, chromoximycin, dactinomycin, daunorubicin, ditrisarubicin B, dexamethasone, doxorubicin, doxorubicin-fibrinogen, elsamicin-A, epirubicin, erbstatin, esorubicin, esperamicin-Al, esperamicin-Alb, fostriecin, glidobactin, gregatin-A, grincamycin, herbimycin, corticosteroids such as hydrocortisone, idarubicin, illudins, kazusamycin, kesarirhodins, menogaril, mitomycin, neoenactin, oxalysine, oxaunomycin, peplomycin, pilatin, pirarubicin, porothramycin, prednisone, prednisolone, pyrindanycin A, rapamycin, rhizoxin, rodorubicin, sibanomicin, siwenmycin, sorangicin-A, sparsomycin, talisomycin, terpentecin, thrazine, tricrozarin A, and zorubicin. The amount of the antibiotic agent administered to a mammal having cancer is an amount that is sufficient to treat the cancer whether administered alone or in combination with a vitamin D compound.

Alternatively, the vitamin D compound may also be used with other anti-cancer agents, including but not limited to, acemannan, aclarubicin, aldesleukin, alemtuzumab, alitretinoin, altretamine, amifostine, amsacrine, anagrelide, anastrozole, ancestim, bexarotene, broxuridine, capecitabine, celmoleukin, cetrorelix, cladribine, clotrimazole, daclizumab, dexrazoxane, dilazep, docosanol, doxifluridine, bromocriptine, carmustine, cytarabine, diclofenac, edelfosine, edrecolomab, eflornithine, emitefur, exemestane, exisulind, fadrozole, filgrastim, finasteride, fludarabine phosphate, formestane, fotemustine, gallium nitrate, gemcitabine, glycopine, heptaplatin, ibandronic acid, imiquimod, iobenguane, irinotecan, irsogladine, lanreotide, leflunomide, lenograstim, lentinan sulfate, letrozole, liarozole, lobaplatin, lonidamine, masoprocol, melarsoprol, metoclopramide, mifepristone, miltefosine, mirimostim, mitoguazone, mitolactol, molgramostim, nafarelin, nartograstim, nedaplatin, nilutamide, noscapine, oprelvekin, osaterone, oxaliplatin, pamidronic acid, pegaspargase, pentosan polysulfate sodium, pentostatin, picibanil, pirarubicin, porfimer sodium, raloxifene, raltitrexed, rasburicase, rituximab, romurtide, sargramostim, sizofiran, sobuzoxane, sonermin, suramin, tasonermin, tazarotene, tegafur, temoporfin, temozolomide, teniposide, tetrachlorodecaoxide, thalidomide, thymalfasin, thyrotropin alfa, topotecan, toremifene, trastuzumab, treosulfan, tretinoin, trilostane, trimetrexate, ubenimex, valrubicin, verteporfin, vinorelbine. The amount of the anti-cancer agent administered to a mammal having cancer is an amount that is sufficient to treat the cancer whether administered alone or in combination with a vitamin D compound.

In another embodiment, the vitamin D compound may be administered in combination with an anti-hypercalcemic agent and an additional anti-cancer agent. Suitable anti-hypercalcemic agents include, but are not limited to, bisphosphonates, such as pamidronate, etidronate, alendronate, tiludronate, risedronate, zoledronic acid, clodronate, and bisphosphonate EB 1053. Suitable anti-cancer agents include, but are not limited to, those anti-cancer agents discussed above. The amount of the anti-hypercalcemic agent and anti-cancer agent administered to a mammal having cancer is an amount that is sufficient to treat the vitamin D-induced hypercalcemia and the cancer, respectively, whether administered alone or in combination with a vitamin D compound.

The vitamin D compound may also be administered or combined with steroids, such as corticosteroids or glucocorticoids. The vitamin D compound and the steroid may be administered in the same or in different compositions. Non-limiting examples of suitable steroids include hydrocortisone, prednisone, dexamethasone. The amount of the steroid administered to a mammal having cancer is an amount that is sufficient to treat the cancer whether administered alone or in combination with a vitamin D compound.

In one embodiment, provided herein are methods and compositions comprising both seocalcitol and a steroid particularly a corticosteroid, or more particularly a glucocorticoid. Steroids within the scope of the disclosure include, but are not limited to, (1) hydrocortisone (cortisol; cyprionate (*e.g*., CORTEF), oral; sodium phosphate injection (HYDROCORTONE PHOSPHATE); sodium succinate (e.g., A-HYDROCORT, Solu-CORTEF); cortisone acetate oral or injection forms, etc.), (2) dexamethasone (e.g., Decadron, oral; Decadron-LA injection, etc.), (3) prednisolone (e.g., Delta-CORTEF, prednisolone acetate (ECONOPRED), prednisolone sodium phosphate (HYDELTRASOL), prednisolone tebutate (HYDELTRA-TBA, etc.)), or (4) prednisone (e.g., DELTASONE, etc.) and combinations thereof. See, e.g., GOODMAN & GILMAN'S THE PHARMACOLOGICAL BASIS OF THERAPEUTICS, 10TH EDITION 2001.

In a specific embodiment, single unit solid oral dosage forms which comprise an amount from about 0.1 µg to about 500 µg of seocalcitol and an amount from about 0.5 mg to about 3.0 mg of a steroid, *e.g.,* glucocorticoid in a single composition, optionally with excipients, carriers, diluents, etc. is contemplated. For instance, the single unit dosage form can comprise about 0.1 µg to about 500 µg of seocalcitol and about 1.0 mg, 1.25 mg, 1.5 mg, or 2.0 mg of a steroid, such as but not limited to corticosteroids or glucocorticoids.

### Administration of a Vitamin D Compound and an Additional Therapeutic Agent

The vitamin D compound and the additional therapeutic agent, such as an anti-cancer agent or a steroid can be administered by any method known to one skilled in the art. In certain embodiments, the vitamin D compound and the additional therapeutic agent can be in separate compositions prior to administration. In the alternative, the vitamin D compound and the additional therapeutic agent can be combined into a single composition for administration.

The vitamin D compound and the additional therapeutic agent can be administered sequentially or simultaneously. If administered sequentially, the order of administration is flexible. For instance, a vitamin D compound can be administered prior to administration of the additional therapeutic agent. Alternatively, administration of the additional therapeutic agent can precede administration of a vitamin D compound.

In certain embodiments, the vitamin D compound is administered by pulse administration or dosing. Examples of pulse dosing of a vitamin D compound are disclosed in U.S. Patent No. 6,521,608 to Henner et al. In certain embodiments, a pulse administration dosing regimen can include a single dose administration of a vitamin D compound followed by one week of non-administration of a vitamin D compound. In other embodiments, a pulse administration dosing regimen can include a single dose administration of a vitamin D compound followed by three weeks of non-administration of a vitamin D compound. In still other embodiments, a pulse administration dosing regimen can include a single dose administration of a vitamin D compound followed by one month of non-administration of a vitamin D compound.

Whether they are administered as separate compositions or in one composition, each composition is preferably pharmaceutically suitable for administration. Moreover, the vitamin D compound and the additional therapeutic agent, if administered separately, can be administered by the same or different modes of administration. Examples of modes of administration include parenteral (*e.g.,* subcutaneous, intramuscular, intraorbital, intracapsular, intraspinal, intrasternal, intravenous, intradermal, intraperitoneal, intraportal, intra-arterial, intrathecal, transmucosal, intra-articular, and intrapleural,), transdermal (*e.g.,* topical), epidural, and mucosal (*e.g.,* intranasal) injection or infusion, as well as oral, inhalation, pulmonary, and rectal administration. In specific embodiments, both are oral.

For example, the vitamin D compound can be administered transdermally and the additional therapeutic agent can be administered parenterally. Alternatively, the vitamin D compound can be administered orally, such as in a tablet, caplet or capsule, while the additional therapeutic agent can be administered intravenously. Such intravenous administered therapeutic agents include, but are not limited to, docetaxel injections, such as Taxotere^{®}; paclitaxel injections, such as Paclitaxel^{®} and mitoxantrone injections, such as Novantrone^{®}. Also, the additional therapeutic agent can be in the form of depots or implants such as leuprolide depots and implants, *e.g.* Viadur^{®} and Lupron Depot^{®}; triptorelin depots, *e.g.* Trelstar^{®}; goserelin implants, *e.g.* Zoladex^{®}.

The suitable daily dosage of the vitamin D compound depends upon a number of factors, including, the nature of the severity of the condition to be treated, the particular inhibitor, the route of administration and the age, weight, and response of the individual patient. Suitable daily dosages of vitamin D compound can generally range from about 0.000001 µg/kg/day to about 125 µg/kg/day, or from about 0.00001 µg/kg/day to about 40 µg/kg/day, or from about 0.0001 µg/kg/day to about 20 µg/kg/day, or from about 0.001 µg/kg/day to about 15 µg/kg/day in single or multiple doses.

In some embodiments, the vitamin D compound can be administered in an amount from about 0.0001 µg/day to about 2,500 µg/day. In certain embodiments, the vitamin D compound can be administered in an amount from about 0.001 µg/day to about 1,000 µg/day or from about 0.01 µg/day to about 750 µg/day or from about 0.1 µg/day to about 500 µg/day in single or multiple doses. In certain embodiments, seocalcitol is administered in single or multiple doses in an amount of about 50 µg/day or about 100 µg/day.

In certain embodiments, the vitamin D compound, such as seocalcitol, is co-administered with an additional anti-cancer agent such as mitoxantrone, paclitaxel or docetaxel. For example, a method for the treatment of a cancer in a mammal comprises administering an amount of seocalcitol and an amount of mitoxantrone. For example, the seocalcitol can be administered in an amount of about 0.1 µg/day to about 500 µg/day, such as about 100 µg/day, and the mitoxantrone can be administered in an amount of about 0.1 mg/m² to about 20 mg/m². Preferably, the mitoxantrone is administered over a period of between about 10 to about 20 minutes once every 21 days.

Also, a method for the treatment of a cancer in a mammal can comprise administering an amount of seocalcitol and an amount of paclitaxel. In one embodiment, the seocalcitol can be administered in an amount of about 0.1 µg/day to about 500 µg/day, such as about 100 µg/day, and the paclitaxel can be administered in the amount of about 1 mg/m² to about 175 mg/m². Preferably, the paclitaxel is administered over a period of between about 2 to about 5 hours once every three months.

Additionally, a method for the treatment of a cancer in a mammal comprises administering an amount of seocalcitol and an amount of docetaxel. For example, the seocalcitol can be administered in an amount of about 0.1 µg/day to about 500 µg/day, such as about 100 µg/day, and the docetaxel can be administered in an amount of about 1 mg/m² to about 100 mg/m². Preferably, the docetaxel is administered over a period of between about 1 to about 2 hours once every three weeks.

In certain embodiments, the vitamin D compound, such as seocalcitol, is administered along with an anti-cancer agent that comprises a hormonal ablation agent, including, but not limited to, leuprolide, goserelin, or triptorelin. For example, one method for the treatment of a cancer in a mammal also comprises administering an amount of seocalcitol and an amount of leuprolide. The amount of seocalcitol can be about 0.1 µg/day to about 500 µg/day, such as about 100 µg/day, and the amount of leuprolide can be about 0.01 mg to about 200 mg over a period of about 3 days to about 12 months. Preferably, the leuprolide is administered in the amount of about 3.6 mg of leuprolide over a period of about 3 days to about 12 months.

Additionally, the methods for the treatment of cancer in a mammal include administering an amount of seocalcitol and an amount of goserelin. For example, the amount of seocalcitol can be about 0.1 µg/day to about 500 µg/day, such as about 100 µg/day, and the amount of goserelin can be about 0.01 mg to about 20 mg over a period of about 28 days to about 3 months. Preferably, the goserelin is administered in the amount of about 3.6 mg to about 10.8 mg over a period of about 28 days to about 3 months.

In certain embodiments, the methods for the treatment of cancer in a mammal comprise administering an amount of seocalcitol and an amount of triptorelin. For example, the amount of seocalcitol can be about 0.1 µg/day to about 500 µg/day, such as about 100 µg/day, and the amount of triptorelin can be about 0.01 mg to about 20 mg, over a period of about 1 month, preferably, the triptorelin is administered in the amount of about 3.75 mg over a period of about 1 month.

Also, in one embodiment, the method for the treatment of a cancer in a mammal comprises administering an amount of seocalcitol and an amount of abiraterone acetate. For instance, the method involves administering an amount of about 0.1 µg/day to about 500 µg/day of seocalcitol, such as about 100 µg/day of seocalcitol, and an amount of about 0.4 mg/day to about 10,000 mg/day of abiraterone acetate.

In another embodiment, the method for the treatment of a cancer in a mammal comprises administering an amount of seocalcitol and an amount of bicalutamide. For instance, the method involves administering an amount of seocalcitol can be about 0.1 µg/day to about 500 µg/day, such as about 100 µg/day, and an amount of about 1 mg/day to about 300 mg/day of bicalutamide.

In yet another embodiment, the method for the treatment of a cancer in a mammal comprises administering an amount of seocalcitol and an amount of flutamide. For example, the method comprises administering an amount of seocalcitol can be about 0.1 µg/day to about 500 µg/day, such as about 100 µg/day, and an amount of about 1 mg/day to about 2000 mg/day of flutamide.

Moreover, the method for the treatment of a cancer in a mammal can comprise administering an amount of a vitamin D compound and an amount of a glucocorticoid, including but not limited to hydrocortisone, prednisone or dexamethasone. For example, the method can comprise administering an amount of about 0.1 µg/day to about 500 µg/day of seocalcitol, such as about 100 µg/day, and an amount of about 0.01 mg/day to about 500 mg/day of hydrocortisone. In other instances, the method can comprise administering an amount of about 0.1 µg/day to about 500 µg/day of seocalcitol and an amount of about 10 mg/day to about 250 mg/day of hydrocortisone.

The method for the treatment of a cancer can also comprise administering an amount of a vitamin D compound and an amount of a glucocorticoid, such as prednisone. For example, the method can comprise administering an amount of about 0.1 µg/day to about 500 µg/day of seocalcitol, such as about 100 µg/day, and an amount of about 0.01 mg/day to about 500 mg/day of prednisone. Also, the method can comprise administering an amount of about 0.1 µg/day to about 500 µg/day of seocalcitol and an amount of about 10 mg/day to about 250 mg/day of prednisone.

In addition, the method for the treatment of a cancer can also comprise administering an amount of a vitamin D compound and an amount of a glucocorticoid, such as dexamethasone. For example, the method can comprise administering an amount of about 0.1 µg/day to about 500 µg/day of seocalcitol, such as about 100 µg/day, and an amount of about 0.01 mg/day to about 500 mg/day of dexamethasone. Also, the method can comprise administering an amount of 0.1 µg/day to about 500 µg/day of seocalcitol and an amount of about 0.5 mg/day to about 25 mg/day of dexamethasone.

In still another embodiment, the method for the treatment of a cancer in a mammal comprises pulse administering an amount of seocalcitol in combination with a chemotherapy treatment.

### Compositions Comprising a Vitamin D Compound and an Additional Therapeutic Agent

In certain embodiments, the compositions can contain a combination of a vitamin D compound, preferably seocalcitol, and any of the therapeutic agents recited above. Whether the vitamin D compound and the additional therapeutic agent are administered in separate compositions or as a single composition, the compositions can take various forms. For example, the compositions can take the form of solutions, suspensions, emulsions, tablets, pills, capsules, powders or sustained-release formulations, depending on the intended route of administration.

For topical or transdermal administration, the compositions can be formulated as solutions, gels, ointments, creams, suspensions or salves.

For oral administration, the compositions may be formulated as tablets, pills, dragees, troches, capsules, liquids, gels, syrups, slurries, suspensions and the like, for oral ingestion by a patient to be treated.

The composition may also be formulated in rectal or vaginal compositions such as suppositories or retention enemas that contain conventional suppository bases such as cocoa butter or other glycerides.

In addition to the formulations described previously, the composition may also be formulated as a depot preparation. Such long acting formulations may be administered by implantation (for example subcutaneously or intramuscularly) or by intramuscular injection. Thus, for example, the therapeutic agents may be formulated with suitable polymeric or hydrophobic materials (for example as an emulsion in an acceptable oil) or ion exchange resins, or as sparingly soluble derivatives, for example, as a sparingly soluble salt.

Additionally, the composition may be delivered using a sustained-release system, such as semi-permeable matrices of solid polymers containing the composition. Various forms of sustained-release materials have been established and are well known by those skilled in the art. Sustained-release capsules may, depending on their chemical nature can release the composition over a period of hours, days, weeks, months. For example, a sustained release capsule can release the compositions over a period of 100 days or longer. Depending on the chemical nature and the biological stability of the composition, additional strategies for stabilization may be employed.

The compositions can further comprise a pharmaceutically acceptable carrier. The term "carrier" refers to a diluent, adjuvant (*e.g.,* Freund's adjuvant (complete and incomplete)), excipient, or vehicle with which the therapeutic is administered.

For parenteral administrations, the composition can comprise one or more of the following carriers: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

For oral solid formulations suitable carriers include fillers such as sugars, e.g., lactose, sucrose, mannitol and sorbitol; cellulose preparations such as maize starch, wheat starch, rice starch, potato starch, gelatin, gum tragacanth, methyl cellulose, hydroxypropylmethyl-cellulose, sodium carboxymethylcellulose, fats and oils; granulating agents; and binding agents such as microcrystalline cellulose, gum tragacanth or gelatin; disintegrating agents, such as cross-linked polyvinylpyrrolidone, agar, or alginic acid or a salt thereof such as sodium alginate, Primogel, or corn starch; lubricants, such as magnesium stearate or Sterotes; glidants, such as colloidal silicon dioxide; a sweetening agent, such as sucrose or saccharin; or flavoring agents, such as peppermint, methyl salicylate, or orange flavoring. If desired, solid dosage forms may be sugar-coated or enteric-coated using standard techniques. Other suitable oral formulations include lipid-containing formulations, such as, but not limited to, medium chain triglyceride and long chain triglyceride. The lipid formulations have been shown to have a positive effect on the solubilization process resulting in a twofold higher bioavailability of seocalcitol compared with a formulation containing propylene glycol. Examples of methods of making suitable lipid formulations are disclosed in Journal of Pharmaceutical Sciences, 94, 1830-1838, 2005, herein incorporated by reference.

For intravenous administration, suitable carriers include physiological saline, bacteriostatic water, phosphate buffered saline (PBS). In all cases, the composition must be sterile and should be fluid to the extent that easy injectability with a syringe. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars; polyalcohols such as mannitol, sorbitol; sodium chloride in the composition. Prolonged absorption of the injectable compositions can be brought about by including in the composition an agent which delays absorption, for example, aluminum monostearate and gelatin.

Also for intravenous administration, the compositions may be formulated in solutions, preferably in physiologically compatible buffers such as Hanks's solution, Ringer's solution, or physiological saline buffer. The solution may contain formulatory agents such as suspending, stabilizing and/or dispersing agents. In a preferred embodiment, the compositions are formulated in sterile solutions.

For transmucosal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories.

For administration by inhalation, the compositions may be formulated as an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Capsules and cartridges of gelatin for use in an inhaler or insufflator may be formulated containing a powder mix of the composition and a suitable powder base such as lactose or starch.

The pharmaceutical compositions may be manufactured by means of conventional mixing, dissolving, granulating, dragee-making, levigating, emulsifying, encapsulating, entrapping or lyophilizing processes.

One example of a composition comprising a vitamin D compound and an additional therapeutic agent is an oral composition or composition suitable for oral administration comprising seocalcitol in combination with a steroid. For example, the oral composition can be a solid dosage form, such as a pill, a tablet or a capsule. The oral composition can comprise about 0.0001 µg, 0.001 µg, 0.01 µg, 0.05 µg, 0.1 µg, 0.25 µg, 0.5 µg, 0.75 µg, 1.0 µg, 2.5 µg, 5.0 µg, 7.5 µg, 10 µg, 25 µg, 50 µg, 75 µg, 100 µg, 150 µg, 200 µg, 250 µg, 300 µg, 350 µg, 400 µg, 450 µg, 500 µg, 550 µg, 600 µg, 650 µg, 700 µg, 750 µg, 800 µg, 850 µg, 900 µg, 950 µg, or 1000 µg of seocalcitol. The oral composition can comprises about 0.25 mg, 0.5 mg, 0.75 mg, 1.0 mg, 1.25 mg, 1.5 mg, 1.75 mg, 2.0 mg, 2.25 mg, 2.5 mg, 2.75 mg, 3.0 mg, 3.25 mg, 3.5 mg, 3.75 mg, 4.0 mg, 4.25 mg, 4.5 mg, 4.75 mg, 5.0 mg, 7.5 mg, 10 mg, 20 mg, 30 mg, 40 mg or 50 mg of a steroid.

In one embodiment, the oral composition can comprise about 0.1 µg to about 500 µg of seocalcitol and an amount of about 0.25 mg to about 3.5 mg of the steroid, such as hydrocortisone, prednisone or dexamethasone. In other instances, the composition can comprise about 0.1 µg to about 500 µg of seocalcitol and an amount of about 1.0 mg to about 2.5 mg of the steroid, such as hydrocortisone, prednisone or dexamethasone.

The description contained herein is for purposes of illustration and not for purposes of limitation. The methods and compositions described herein can comprise any feature described herein either alone or in combination with any other feature(s) described herein. Changes and modifications may be made to the embodiments of the description. Furthermore, obvious changes, modifications or variations will occur to those skilled in the art. Also, all references cited above are incorporated herein, in their entirety, for all purposes related to this disclosure.

The invention also encompasses the following embodiments
1. A method for the treatment of a prostate cancer or a breast cancer in a mammal comprising administering a therapeutically effective amount of a vitamin D compound and a therapeutically effective amount of at least one additional therapeutic agent to a patient having a prostate cancer or a breast cancer.
2. The method of embodiment 1, wherein the vitamin D compound comprises seocalcitol.
3. The method of embodiment 1, wherein the therapeutically effective amount of the vitamin D compound comprises about 0.001 µg/day to about 1000 µg/day.
4. The method of embodiment 1, wherein the additional therapeutic agent comprises an anti-neoplastic agent, an alkylating agent, an anti-metabolite agent, an antibiotic agent, a hormonal ablation agent, an androgen ablation agent, an anti-androgen agent, or a steroid.
5. The method of embodiment 1, wherein the vitamin D compound and the additional therapeutic agent are administered to the mammal in a single composition comprising the vitamin D compound and the additional therapeutic agent.
6. The method of embodiment 1, wherein the vitamin D compound and the additional therapeutic agent are administered separately to the mammal.
7. The method of embodiment 1, wherein the vitamin D compound is administered by pulse administration.
8. The method of embodiment 1, wherein the method further comprises administering an anti-hypercalcemic agent.
9. The method of embodiment 1, wherein the vitamin D compound comprises seocalcitol and the additional therapeutic agent comprises mitoxantrone, paclitaxel, docetaxel, leuprolide, goserelin, triptorelin, abiraterone acetate, bicalutamide, flutamide, hydrocortisone, prednisone or dexamethasone.
10. The method of embodiment 9, wherein the therapeutically effective amount of the seocalcitol comprises about 0.1 µg/day to about 500 µg/day and the therapeutically effective amount of the additional therapeutic agent comprises about 0.1 mg/m² to about 20 mg/m² of mitoxantrone.
11. The method of embodiment 9, wherein the therapeutically effective amount of the seocalcitol comprises about 0.1 µg/day to about 500 µg/day and the therapeutically effective amount of the additional therapeutic agent comprises about 1 mg/m² to about 175 mg/m² of paclitaxel.
12. The method of embodiment 9, wherein the therapeutically effective amount of the seocalcitol comprises about 0.1 µg/day to about 500 µg/day and the therapeutically effective amount of the additional therapeutic agent comprises about 1 mg/m² to about 100 mg/m² of docetaxel.
13. The method of embodiment 9, wherein the therapeutically effective amount of the seocalcitol comprises about 0.1 µg/day to about 500 µg/day and the therapeutically effective amount of the additional therapeutic agent comprises about 0.01 to about 200 mg of leuprolide over a period of about 3 days to about 12 months.
14. The method of embodiment 9, wherein the therapeutically effective amount of the seocalcitol comprises about 0.1 µg/day to about 500 µg/day and the therapeutically effective amount of the additional therapeutic agent comprises about 0.01 mg to about 20 mg of goserelin over a period of about 28 days to about 3 months.
15. The method of embodiment 9, wherein the therapeutically effective amount of the seocalcitol comprises about 0.1 µg/day to about 500 µg/day and the therapeutically effective amount of the additional therapeutic agent comprises about 0.01 mg to about 20 mg of triptorelin over a period of about 1 month.
16. The method of embodiment 9, wherein the therapeutically effective amount of the seocalcitol comprises about 0.1 µg/day to about 500 µg/day and the therapeutically effective amount of the additional therapeutic agent comprises about 0.4 mg/day to about 10,000 mg/day of abiraterone acetate.
17. The method of embodiment 9, wherein the therapeutically effective amount of the seocalcitol comprises about 0.1 µg/day to about 500 µg/day and the therapeutically effective amount of the additional therapeutic agent comprises about 1 mg/day to about 300 mg/day of bicalcutamide.
18. The method of embodiment 9, wherein the therapeutically effective amount of the seocalcitol comprises about 0.1 µg/day to about 500 µg/day and the therapeutically effective amount of the additional therapeutic agent comprises about 1 mg/day to about 2000 mg/day flutamide.
19. The method of embodiment 9, wherein the therapeutically effective amount of the seocalcitol comprises about 0.1 µg/day to about 500 µg/day and the therapeutically effective amount of the additional therapeutic agent comprises about 10 mg/day to about 250 mg/day of hydrocortisone or prednisone.
20. The method of embodiment 9, wherein the therapeutically effective amount of the seocalcitol comprises about 0.1 µg/day to about 500 µg/day and the therapeutically effective amount of the additional therapeutic agent comprises about 0.5 mg/day to about 25 mg/day dexamethasone.
21. A method for treating a patient having refractory prostate cancer or refractory breast cancer who is currently receiving at least one treatment for cancer, the method comprising administering a therapeutically effective amount of a vitamin D compound in addition to the at least one treatment the patient is currently receiving.
22. The method of embodiment 21, wherein the vitamin D compound comprises seocalcitol.
23. The method of embodiment 21, wherein the therapeutically effective amount of the vitamin D compound comprises about 0.001 µg/day to about 1000 µg/day,
24. The method of embodiment 21, wherein the treatment for cancer comprises the administration of mitoxantrone, paclitaxel, docetaxel, leuprolide, goserelin, triptorelin, abiraterone acetate, bicalutamide, flutamide, hydrocortisone, prednisone or dexamethasone.
25. A pharmaceutical composition for the treatment of a cancer in a mammal comprising a therapeutically effective amount of seocalcitol and a therapeutically effective amount of a steroid, wherein the composition is suitable for oral administration.
26. The composition of embodiment 5, wherein the composition comprises about 0.1 µg to about 500 µg of seocalcitol and about 0.25 mg to about 3.5 mg of the steroid.
27. The composition of embodiment 25, wherein the steroid comprises hydrocortisone, prednisone or dexamethasone.
28. The composition of embodiment 25, wherein the composition is in the form of a pill, tablet or capsule.

## Claims

1. A pharmaceutical composition comprising a vitamin D compound and an additional therapeutic agent for use in a method of treating a breast cancer.

2. A product containing a vitamin D compound and an additional therapeutic agent as a combined preparation for simultaneous, separate or sequential use in a method of treating a breast cancer.

3. A vitamin D compound for use in a method of treating a breast cancer, said method comprising administering the vitamin D compound in combination with at least one additional therapeutic agent; or a therapeutic agent for use in a method of treating a breast cancer, said method comprising administering the therapeutic agent in combination with a vitamin D compound.

4. Use of a vitamin D compound in the manufacture of a medicament for a method of treating breast cancer, wherein the vitamin D compound is administered in combination with at least one additional therapeutic agent; or use of a therapeutic agent in the manufacture of a medicament for a method of treating breast cancer, wherein the therapeutic agent is administered in combination with a vitamin D compound.

5. The pharmaceutical composition, the product, the vitamin D compound, the therapeutic agent or the use as claimed in claims 1 to 4, wherein the therapeutic agent comprises an anti-neoplastic agent, an alkylating agent, an anti-metabolite agent, an antibiotic agent, a hormonal ablation agent, an androgen ablation agent, an anti-androgen agent or a steroid.

6. The product, the vitamin D compound, the therapeutic agent or the use as claimed in claims 2 to 4, wherein the vitamin D compound and the additional therapeutic agent are administered in a single composition comprising the vitamin D compound and the additional therapeutic agent.

7. The product, the vitamin D compound, the therapeutic agent or the use as claimed in claims 2 to 4, wherein the vitamin D compound and the additional therapeutic agent are administered separately.

8. The pharmaceutical composition, the product, the vitamin D compound, the therapeutic agent or the use as claimed in claims 1 to 4, wherein the vitamin D compound comprises seocalcitol and the therapeutic agent comprises mitoxantrone, paclitaxel, docetaxel, leuprolide, goserelin, triptorelin, abiraterone acetate, bicalutamide, flutamide, hydrocortisone, prednisone or dexamethasone.

9. The pharmaceutical composition, the product, the vitamin D compound, the therapeutic agent or the use as claimed in claim 8, wherein amount of seocalcitol comprises about 0.1µg/day to about 500µg/day and the amount of the therapeutic agent comprises:
a. about 0.1mg/m² to about 20mg/m² of mixantrone, or
b. about 1 mg/m² to about 175mg/m² of paclitaxel, or
c. about 1 mg/m² to about 100mg/m² of docetaxel, or
d. about 0.01 to about 200mg of leuprolide over a period of about 3 days to about 12 months, or
e. about 0.01 mg to about 20mg of goserelin over a period of about 28 days to about 3 months, or
f. about 0.01 mg to about 20mg of triptorelin over a period of about 1 month, or
g. about 0.4mg/day to about 10,000mg/day of abiraterone acetate, or
h. about 1 mg/day to about 300mg/day of bicalcutamide, or
i. about 1 mg/day to about 2000mg/day of flutamide, or
j. about 10mg/day to about 250mg/day of hydrocortisone or prednisone, or
k. about 0.5mg/day to about 25mg/day dexamethasone.

10. A vitamin D compound for use in a method of treating a refractory breast cancer in a patient who is currently receiving at least one treatment for cancer, the method comprising administering the vitamin D compound in addition to at least one treatment the patient is currently receiving.

11. Use of a vitamin D compound in the manufacture of a medicament for a method of treating refractory breast cancer in a patient who is currently receiving at least one treatment for cancer, the method comprising administering the vitamin D compound in addition to at least one treatment the patient is currently receiving.

12. The pharmaceutical composition, the product, the vitamin D compound, the therapeutic agent or the use as claimed in claims 1 to 4, 10 and 11, wherein the vitamin D compound comprises seocalcitol.

13. The pharmaceutical composition, the product, the vitamin D compound, the therapeutic agent or the use as claimed in claims 1 to 4, 10 and 11, wherein the vitamin D compound comprises about 0.001µg/day to about 1000µg/day.

14. The vitamin D compound or the use as claimed in claim 10 and 11, wherein the treatment for cancer comprises the administration of mitoxantrone, paclitaxel, docetaxel, leuprolide, goserelin, triptorelin, abiraterone acetate, bicalutamide, flutamide, hydrocortisone, prednisone or dexamethasone.

15. The pharmaceutical composition according to claim 1 wherein the vitamin D compound is seocalcitol and the additional therapeutic agent is a steroid, and wherein the composition is suitable for oral administration.
